# EUROPÄISCHE PATENTANMELDUNG

(11) **EP 0 537 385 A1**
(43) Veröffentlichungstag der Anmeldung: **21.04.1993**
(21) Anmeldenummer: 91250286.1
(22) Anmeldetag: 14.10.1991
(51) Int. Cl.: A61B 5/12, A61F 11/00, A61N 2/02, H04R 25/00

(54) **Einrichtung zur Tinnitus-Therapie**

(71) Anmelder: DORN GmbH, D-22045 Hamburg (DE)
(72) Erfinder: Lucks, Matthias, D-2000 Hamburg 60 (DE); Bick-Sander, Winfried, D-2000 Hamburg 70 (DE); Giessler, Reiner, D-2000 Hamburg 76 (DE)

(57) **Zusammenfassung**

Eine leicht zu handhabende Einrichtung (1) zur Linderung des Tinitus umfaßt einen stromdurchlossenen Leiter (2) und einen Knochenhörer (3), wobei der stromduchflossene Feld beaufschlagt und gleichzeitig der Knochenhörer den Mastoid-Bereich mit einem Ton und/oder Rauschen bestimmter Frequenz und Lautstärke beschallt. Durch die Einrichtung, welche mit vernünftigem Aufwand hergestellt werden kann, entfällt die Belastung des Patienten durch die von hohen Medikamenten-Dosen hervorgerufenen Nebenwirkungen.

## Beschreibung

Die Erfindung betrifft eine Einrichtung zur Behandlung des menschlichen Gehörs durch Tinnitus-Therapie.

Es sind zahlreiche therapeutische Methoden bekannt, mit denen ein Tinnitus-Patient behandelt werden kann. Prinzipiell unterscheidet man in physikalische, medikamentöse, Psycho- und chirurgische Therapie. Zu den physikalischen Anwendungen gehören die sogenannten Masker, bei denen der Tinnitus durch einen von außen zugeführten Ton bzw. Rauschen über- bzw. verdeckt wird. Das Maskierungsgeräusch muß dabei breitbändiger als das zu verdeckende Ohrgeräusch sein. Weiterhin gehört die Elektrostimulation zu den physikalischen Therapien des Tinnitus. Dabei ruft eine innenohrnahe Kathode bei entsprechenden Spannungen und Strömen eine Hörempfindung hervor, oder durch Einsatz einer Anode werden die Ohrgeräusche vermindert oder deren Charakter verändert. Pulsierende Stimulation und modulierter Wechselstrom sollen bei diesem Verfahren wirkungsvoller sein als Gleichstromreizung. Der Wirkungsmechanismus ist allerdings ungeklärt. Es wird eine endocochleäre oder in den Nervenfasern der zentralen Hörbahn gelegene Hyperpolarisation angenommen, welche deren Spontanaktivität vermindert. Eine weitere physikalische Therapie stellt die Akupunktur und Elektroakupunktur dar. Die medikamentöse Therapie erfolgt durch sogenannte durchblutungsfördernde Medikamente. Hier sind jedoch häufig bei unbedenklichen Dosen keine zufriedenstellenden, die Ohrgeräusche zuverlässig vermindernden Wirkungen zu erreichen. Bei hohen Medikamentdosierungen muß die Gefahr von unerwünschten Nebenwirkungen wie Übelkeit, Benommenheit und Schwindel in Kauf genommen werden. Zudem ist die Wirkung oftmals nur von kurzer Dauer (Minuten bis Stunden). Auch die psychotherapeutische Behandlung durch ein sogenannten Entspannungs-Biofeedback ist oftmals nicht zufriedenstellend. Schließlich ist beim absoluten Versagen aller vorher genannten Verfahren eine chirurgische Therapie möglich, worunter eine zerstörende Operation am Innenohr wie Labyrinthektomien und Neurektomien des N. vestibulocochlearis zu verstehen ist. Alle diese Therapie-Methoden sind letztlich unbefriedigend, weil sie keine durchgreifende und dauerhafte Heilung des Patienten vom Tinnitus gewährleisten

Der Erfindung liegt nach allem die Aufgabe zugrunde, eine in der Applikation leicht zu handhabende Einrichtung zur Therapie des Tinnitus zu schaffen, die eine vollständige Heilung, zumindest aber eine deutliche Linderung des Tinnitus mit einfachen physikalischen Maßnahmen ermöglicht.

Diese Aufgabe wird dadurch gelöst, daß die Einrichtung einen Stromdurchflossenen Leiter und einen Knochenhörer umfaßt, wobei der Stromdurchflossene Leiter mindestens einen Mastoid-Bereich mit einem elektromagnetischen Feld beaufschlagt und gleichzeitig der Knochenhörer den Mastoid-Bereich mit einem Ton und/oder Rauschen bestimmter Frequenz und Lautstärke beschallt. Es hat sich herausgestellt, daß eine solche Einrichtung bei vernünftigem Aufwand bezüglich der Herstellung eine zuverlässig funktionierende Therapie des Tinnitus ermöglicht. Die Behandlung eines Tinnitus-Patienten mit dieser Einrichtung ist problemlos durchzuführen und einfach zu überwachen. Eine Belastung des Patienten durch von hohen Medikamenten-Dosen hervorgerufene Nebenwirkungen entfällt Vollständig.

Vorteilhafterweise kann der Stromdurchflossene Leiter von einer Spule gebildet sein, und der vom Knochenhörer erzeugte Ton und/oder das von ihm erzeugte Rauschen kann der Frequenz und mindestens der Lautstärke des Tinnitus entsprechen. Durch die Ausbildung des stromdurchflossenen Leiters als Spule ist die Erzeugung eines den jeweiligen Bedingungen genügenden elektromagnetischen Feldes möglich, wobei ggf. die Spule über einen Transformator zur Erzeugung von Gleichstrom bei 6V betrieben werden kann, so daß an der Spule eine Spannung von 0,9V anliegt. Der vom Knochenhörer erzeugte Ton bzw. das von ihm erzeugte Rauschen entsprechen den während einer Vorbehandlung festgestellten , nach subjektiven Angaben des Patienten gemachten Tönen bzw. dem Rauschen des Tinnitus mit der jeweils entsprechenden Frequenz und Lautstärke. Somit wird eine besonders erfolgreiche Therapie des Tinnitus erreicht.

Vorteilhafterweise kann die Lautstärke des vom Knochenhörer erzeugten Tones und/oder Rauschens bis zu 15 dB über der Lautstärke des Tinnitus liegen. Damit läßt sich eine bezüglich des angestrebten Heilungseffektes besonders zuverlässige Therapie des Tinnitus erreichen.

Eine besonders herstellungsgünstige Einrichtung nach der Erfindung ist dadurch erreicht, daß Knochenhörer und Spule als integraler Bestandteil eines Gerätes nach Art eines Kopfhörers ausgebildet sind und elektromagnetisches Feld und Töne und/oder Rauschen kurzzeitig und impuls- oder schußartig erzeugt werden. Ein so ausgebildetes Gerät ist besonders einfach und günstig zu bedienen.

Weitere Vorteile und Ausführungsformen oder -möglichkeiten der Erfindung gehen aus der folgenden Beschreibung der in der schematischen Zeichnung dargestellten Ausführungsbeispiele hervor. Es zeigt
- Fig. 1: eine Vorderansicht auf eine erfindungsgemäße Einrichtung zur Therapie der Tinnitus und
- Fig. 2: eine Seitenansicht dieser Einrichtung.

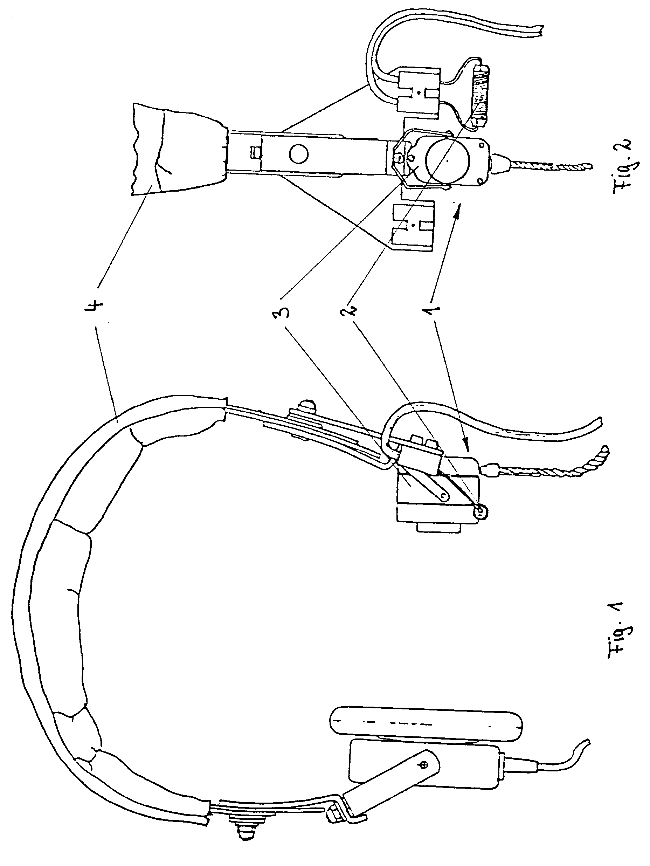

Vor der Therapie wird der Tinnitus nach den subjektiven Angaben des Patienten festgelegt. Dabei werden dem Patienten Töne oder Rauschen in unterschiedlichen Frequenzen und Lautstärken über Kopfhörer vorgespielt. Entspricht der Ton in Frequenz und Lautstärke dem Tinnitus des Patienten, werden die Frequenz in Hz und die Lautstärke in dB festgehalten. Dann wird eine erfindungsgemäße Einrichtung 1 in Form eines Gerät 4 nach Art eines Kopfhörers dem Patienten so aufgesetzt, daß ein Knochenhörer 3 und eine Spule 2 auf dem Knochen, in dem sich das Sinnesorgan befindet, also dem Mastoid-Bereich, aufliegen. In der vorher festgestellten Frequenz wird dieser Bereich nun über den Knochenhörer 3 schwellennah beschallt. Die Lautstärke kann bis zu 15dB über der Lautstärke des Tinnitus liegen. Gleichzeitig wird über die Spule 2 ein elektromagnetisches Feld erzeugt. Die therapeutische Wirkung ergibt sich aus der Beschallung mit dem Knochenhörer 3 und dem von der Spule 2 erzeugten elektromagnetischen Feld, wobei das Magnetfeld den Mastoid-Bereich schußartig über Sekunden in einer vom menschlichen Körper verkraftbaren Feldstärke beaufschlagt.

## Patentansprüche

1. Einrichtung zur Therapie des Tinnitus **dadurch gekennzeichnet,** daß die Einrichtung (1) einen stromdurchflossenen Leiter (2) und einen Knochenhörer (3) umfaßt, wobei der stromdurchflossene Leiter (2) mindestens einen Mastoid-Bereich mit einem elektromagnetischen Feld beaufschlagt und gleichzeitig der Knochenhörer (3) den Mastoid-Bereich mit einem Ton und/oder Rauschen bestimmter Frequenz und Lautstärke beschallt.

2. Einrichtung nach Anspruch 1, **dadurch gekennzeichnet,** daß der stromdurchflossene Leiter (2) von einer Spule gebildet ist und der vom Knochenhörer (3) erzeugte Ton und/oder das von ihm erzeugte Rauschen der Frequenz und mindestens der Lautstärke des Tinnitus entspricht.

3. Einrichtung nach einem der Ansprüche 1 oder 2, **dadurch gekennzeichnet,** daß die Lautstärke des vom Knochenhörer (3) erzeugten Tones und/oder Rauschens bis zu 15 dB über der Lautstärke des Tinnitus liegt.

4. Einrichtung nach einem der Ansprüche 1 bis 3, **dadurch gekennzeichnet,** daß Knochenhörer (3) und Spule (2) als integraler Bestandteil eines Gerätes (4) nach Art eines Kopfhörers ausgebildet sind und elektromagnetisches Feld und Töne und/oder Rauschen kurzzeitig und impuls- oder schußartig erzeugt werden.
